# FASCICULE DE BREVET EUROPEEN

(11) **EP 0 369 836 B1**
(45) Date de publication et mention de la délivrance du brevet: **10.03.1993**
(21) Numéro de dépôt: 89402808.3
(22) Date de dépôt: 11.10.1989
(51) Int. Cl.: G01N 33/52, G01N 33/545, G01N 33/548, C12Q 1/54, B01L 3/00

(54) **Dispositif à usage unique pour tests biologiques**
Vorrichtung für biologische Tests zum einmaligen Gebrauch
Single use device for biological tests

(30) Priorité: 11.10.1988 FR 8813668
(43) Date de publication de la demande: 23.05.1990
(73) Titulaire: INSTITUT TEXTILE DE FRANCE, 92223 Bagneux Cédex (FR); PROBIOTEC (SARL), 69600 Oullins (FR)
(72) Inventeur: Gavet, Louis, F-69005 Lyon (FR); Chatelin, Roger, F-69380 Lissieu par Lozanne (FR); Sotton, Michel, F-69450 St Cyr au Mont d'Or (FR); Guigal, Robert, F-69004 Lyon (FR); Laurent, Philippe, F-69600 Oullins (FR)
(74) Mandataire: Descourtieux, Philippe

(56) Documents cités:
- EP-A- 0 128 422
- EP-A- 0 183 991
- EP-A- 0 194 578
- EP-A- 0 276 152
- US-A- 4 415 665
- US-A- 4 558 013

## Description

La présente invention concerne des dispositifs à usage unique pour la réalisation de tests biologiques , y compris biochimiques, alimentaires par l'immobilisation de molécules déterminées contenues dans le milieu liquide à tester. Ces dispositifs sont destinés notamment au dosage des molécules déterminées par exemple dans un prélèvement de sang.

On connaît déjà des dispositifs de ce type qui ont pour buts de simplifier le travail des laboratoires et d'éviter les risques de contamination et d'erreurs de dosage. En particulier un dispositif à usage unique pour tests biologiques est décrit dans la demande de brevet internationale PCT/US 85.00870 (WO-A-8 505 451). Ce dispositif comprend d'une part un élément d'immobilisation des molécules, poreux et auquel est lié un capteur des molécules à immobiliser et d'autre part un matériau absorbant en contact avec la surface inférieure de l'élément d'immobilisation. Le fonctionnement de ce dispositif connu est le suivant. Le milieu à tester, qui contient les molécules à doser, est versé sur la surface supérieure de l'élément d' immobilisation , diffuse à travers celui-ci et par capillarité dans le matériau absorbant. Les molécules à doser sont retenues par le capteur sur l'élément d'immobilisation. Ainsi l'élément d'immobilisation filtre en quelque sorte les molécules à doser, et le matériau absorbant a pour rôle d'accélérer l'écoulement du milieu à tester à travers ce filtre.

Or on a trouvé et c'est ce qui fait l'objet de l'invention un nouveau dispositif à usage unique pour tests biologiques. Ce dispositif comporte au moins un élément d'immobilisation de molécules déterminées contenues dans un volume déterminé d'un milieu liquide. Selon l'invention, l'élément d'immobilisation est sous la forme d'un matériau hydrophile fixé sur la surface d'un matériau plan hydrophobe, et le dispositif comprend :
a. une plaque de fond supportant le matériau plan hydrophobe
b. au moins une cavité pour le milieu liquide à tester,dont l'orifice d'évacuation est placé au-dessus du matériau plan hydrophobe
c. et un moyen de raclage agissant sur le matériau hydrophile et apte à déplacer le liquide que celui-ci contient sur la surface du matériau plan hydrophobe.

Ainsi contrairement au dispositif connu, il n'y a pas passage du milieu à tester à travers un élément d'immobilisation poreux, jouant le rôle d'un filtre ; et il n'est plus nécessaire de mettre en oeuvre un matériau absorbant pour accélérer le passage du milieu à travers ce filtre. Selon l'invention, le milieu à tester contenu dans la cavité est déposé sur le matériau hydrophile, capable d immobiliser les molécules déterminées ; il est retenu sur ce matériau éventuellement pendant un temps prédéterminé, que les molécules déterminées soient immobilisées en proportion suffisante ; puis il est extrait et évacué du matériau hydrophile par raclage sur la surface du matériau plan hydrophobe.

Selon une version préférée de l'invention, l'élément d'immobilisation des molécules déterminées se présente sous la forme de fibres hydrophiles, implantées sensiblement verticalement dans le matériau plan hydrophobe. Par exemple, les fibres sont à base de cellulose et le matériau hydrophobe à base de polychlorure de vinyle.

Selon une première version du dispositif selon l'invention, il comporte un seul élément d'immobilisation, une première cavité pour le milieu liquide à tester et au moins une seconde cavité pour un réactif, les cavités étant placées au-dessus du matériau plan hydrophobe de telle sorte que, par un déplacement relatif des cavités et du matériau plan hydrophobe, le matériau hydrophile puisse se trouver successivement sous l'orifice d'évacuation de la première puis de la seconde cavité et le moyen de raclage agit sur le matériau hydrophile après passage de celui-ci sous la première cavité. Dans ce cas le réactif contenu dans la seconde cavité est déposé sur le matériau hydrophile en vue de révéler la présence des molécules qui y sont immobilisées et éventuellement de réaliser leur dosage.

Selon un premier mode de réalisation de cette première version du dispositif de l'invention ,la plaque de fond, les cavités et le moyen de raclage sont assemblés sous forme d'un boîtier à l'intérieur duquel le matériau plan hydrophobe peut se déplacer par glissement sur la plaque de fond.

Selon un second mode de réalisation de cette première version du dispositif de l'invention d'une part la plaque de fond, d'autre part les cavités et le moyen de raclage forment deux ensembles indépendants et le dispositif comprend un mécanisme d'entraînement de l'un ou de l'autre de ces deux ensembles. Dans ce cas le déplacement relatif de l'élément d'immobilisation par rapport aux cavités n'est plus manuel mais automatique du fait du mécanisme d'entraînement.

Dans une variante de ce second mode de réalisation , le dispositif a une forme globalement circulaire et le mécanisme d'entraînement entraîne l'ensemble comprenant les cavités et le moyen de raclage en rotation autour de son axe au-dessus de l'ensemble comprenant la plaque de fond.

Selon une seconde version du dispositif selon l'invention, il comporte une cavité pour le milieu liquide à tester et au moins deux éléments d'immobilisation , chacun pour l'immobilisation de molécules différentes, le premier élément étant placé sous la cavité contenant le milieu liquide à tester et le second élément étant éloigné du premier d'un intervalle donné, et le moyen de raclage est apte à déplacer le liquide, contenu dans le matériau hydrophile du premier élément d'immobilisation , sur la surface du matériau plan hydrophobe le long de l'intervalle donné jusqu'au matériau hydrophile du second élément d'immobilisation.

L'invention sera mieux comprise et d'autres caractéristiques et avantages apparaîtront à la lecture de la description qui va être faite de trois modes de réalisation de l'invention, illustrés par le dessin annexé dans lequel :
Les figures 1 et 2 correspondent à un premier mode de réalisation de la première version d'un dispositif purement manuel, la figure 1 montrant la bande supportant l'élément d'immobilisation, et la figure 2 étant une vue schématique en coupe longitudinale du dispositif.
La figure 3 correspond à un second mode de réalisation de la première version d'un dispositif à fonctionnement automatique et est une vue schématique de dessus du dispositif.
La figure 4 correspond à la seconde version d'un dispositif purement manuel et est une vue schématique en coupe longitudinale du dispositif en cours d'utilisation.

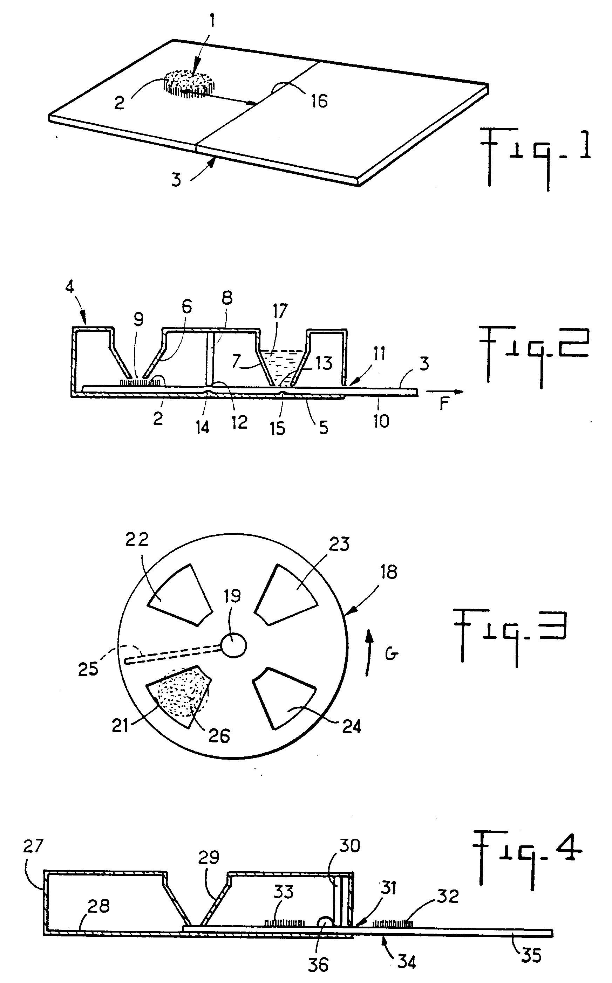

Dans sa forme préférée, l'élément 1 d'immobilisation des molécules déterminées se présente sous forme de fibres 2 implantées par la technique du flocage dans un élément étroit ou bandelette .Sur la figure 1 l'implantation des fibres 2 est circulaire, dans une zone extrême de la bandelette 3. Les fibres 2 sont dans une matière hydrophile, notamment cellulosique ; la bandelette 3 est dans une matière hydrophobe, par exemple en polychlorure de vinyle.

Il est rappelé que le flocage est une technique usuelle dans l'industrie textile. Elle consiste par exemple à projeter des fibres , coupées à une longueur déterminée, dans un support à l'état pâteux, en sorte que la base desdites fibres soit ancrée verticalement dans le support après rigidification de celui-ci. Dans le cas présent il s'agissait d'une bandelette 3 de polychlorure de vinyle de 0,9 mm , et de fibres 2 de viscose ayant un titrage moyen de 1,7 dtex ; les fibres 2 dépassaient de la bandelette 3 sur une hauteur de 0,4 mm.

La figure 2 montre comment est placée la bandelette 3 dans le support 4 avant utilisation . Ce support 4 est une boîte creuse composée des éléments principaux suivants : la partie inférieure qui est une plaque 5 rigide et plane ; la partie supérieure, c'est-à-dire la partie qui vient au-dessus de la bandelette 3, est creusée de deux cavités 6 et 7 et comprend une lame rigide 8, intérieure et placée entre les deux cavités 6 et 7.

Le positionnement de la bandelette 3 à l'intérieur du support 4 et avant utilisation est le suivant : la face inférieure de la bandelette 3 repose sur la plaque de fond 5 ; la zone extrême de la bandelette 3 où se trouve implantée la pastille circulaire 1 de fibres 2 est placée sous la cavité 6 de telle sorte que l'orifice 9 de la cavité 6 soit juste au-dessus de ladite pastille 1 ; la zone extrême 10 de la bandelette opposée à la précédente dépasse du support 4 par une fente 11. La lame 8 , placée immédiatement après la cavité 6, a un bord rectiligne 12 qui s'applique sur la face supérieure de la bandelette 3. L'orifice 13 de la cavité 7 s'applique sur la face supérieure de la bandelette 3 entre la lame 8 et la fente de sortie 11.

La plaque de fond 5 peut comporter en regard du bord 12 de la lame 8 et de l'orifice 13 de la seconde cavité 7 des surépaisseurs 14 et 15.

Par souci de simplification, on a décrit un support avec deux cavités mais le dispositif peut en comprendre un nombre plus élevé , qui est fonction du nombre de réactifs nécessaires pour effectuer le dosage.

Les fibres 2 sont préalablement traitées en sorte qu'elles immobilisent les molécules déterminées, en fonction du dosage à effectuer, par exemple par greffage de sites actifs capables d'interagir avec lesdites molécules par échange d'ions ou par formation de liaisons covalentes.

Dans le cas de dosage d'immunoglobulines dans le sérum humain, on a greffé les fibres 2 de viscose, préalablement à leur implantation dans la bandelette 3 , en leur faisant subir successivement une imprégnation contrôlée d'une solution aqueuse à 20 % de 2 acrylamido 2 méthyl propane sulfonate, une irradiation sous faisceau d'électron à une dose de 2 Mrad, suivie d'un rinçage et d'un séchage. La structure cellulosique de la viscose comporte des greffons porteurs de fonctions sulfoniques.

Pour effectuer le dosage, on introduit dans la première cavité 6 un volume déterminé du milieu biologique à tester, par exemple du sérum. Les fibres 2 hydrophiles absorbent totalement le volume en question. Les fonctions sulfoniques supportées par les greffons de la viscose greffée immobilisent par interaction ionique les protéines qui se trouvent dans le sérum à l'état de macroions. La cinétique de fixation des protéines et la proportion de protéines fixées sont variables en fonction du sérum et du pH. On a remarqué qu'après un temps de contact de cinq minutes, 90 % des immunoglobulines humaines (IgG, IgM, IgA) étaient immobilisées ,quel que soit le pH. La concentration en protéines totale a été mesurée en utilisant la méthode dite de Bradford ; il s'agissait de 350 à 400 mg de protéines dans 0,2 ml de milieu réactionnel.

Après un temps suffisant pour que les molécules déterminées soient immobilisées sur les fibres 2 , on fait coulisser la bandelette 3 sur la plaque de fond 5 en tirant sur la partie extrême 10, dépassant du support 4, jusqu'à ce que le repère 16, marqué sur la face supérieure de la bandelette 3, soit au niveau de la fente 11. Ce repère 16 est par exemple une ligne transversale, éloignée du centre de la pastille circulaire 1 des fibres 2 d'une distance h correspondant à l'écartement entre l'orifice 13 de la seconde cavité 7 et la fente 11. Ainsi lorsque le repère 16 est au droit de la fente 11, la pastille 1 est sous l'orifice 13 de la seconde cavité 7.

Lors du déplacement de la bandelette 3, dans le sens de la flèche F sur la figure 2, les fibres 2 passent entre le bord 12 inférieur de la lame 8 et la plaque de fond 5 ; la lame 8 joue le rôle de racle, comprime les fibres 2 et extrait mécaniquement le liquide qu'elles contiennent. Le liquide extrait glisse sur la surface hydrophobe de la bandelette 3 et reste dans la partie creuse du support comprise entre la cavité 6 et la lame 8.

Le déplacement de la bande se poursuit jusqu'à ce que la pastille 1 soit sous la seconde cavité 7.

Le réactif 17 présent ou introduit dans la seconde cavité 7 est absorbé par les fibres hydrophiles 2, exemptes de liquide , et réagit avec les molécules immobilisées.

On comprend qu'il est nécessaire d'extraire des fibres 2 le liquide initial afin que lesdites fibres puissent absorber de nouveau le réactif 17. Bien sûr, s'agissant d'une extraction purement mécanique, les fibres 2 ne sont pas à proprement parler sèches, elles contiennent l'eau retenue par liaison hydrogène et par capillarité , mais elles ont encore une grande capacité d'absorption.

La présence des surépaisseurs 14 et 15 sur la plaque de fond 5, en regard respectivement de la lame 8 et de la seconde cavité 7, permet d'améliorer entre autres l'étanchéité du dispositif . Le matériau plan hydrophobe constituant la bandelette 3 est de préférence un matériau ayant une certaine élasticité, ce qui est le cas du polychlorure de vinyle. Les surépaisseurs 14 et 15 compriment légèrement la bandelette 3 lorsqu'elle est au droit de la lame 8 et de la seconde cavité 7.

D'une part , l'effet de raclage de la lame 8 se combine à un effet d'exprimage par compression ; d'autre part, la bandelette 3 est constamment appliquée sur les lèvres de l' orifice 13 de la seconde cavité 7. Ainsi , il est possible de stocker dans le dispositif avant utilisation le réactif 17 dans la cavité 7 sans que celui-ci ne s'écoule à l'intérieur du support 4, la fermeture étanche de ladite cavité 7 étant assurée par l'application de la bandelette hydrophobe 3 sur l'orifice 13.

Ainsi , avant usage, le dispositif se présente sous la forme d'un boîtier à l'intérieur duquel est positionnée la bandelette 3 avec la languette 10 dépassant de l'ouverture 11, et dont les secondes cavités 7 sont remplies des réactifs 17 et fermées par scellage d'un film plastique.

Dans le second mode de réalisation de l'invention, illustré par la figure 3, le dispositif a une forme globalement circulaire; la partie supérieure, comportant les cavités pour le milieu à tester et les réactifs , est mobile par rapport à la plaque de fond, selon un mouvement de rotation autour de l'axe 19 dans le sens de la flèche G. Le dispositif 18 représenté à la figure 3 a quatre cavités, la première 21 destinée à l'introduction du milieu à tester, les autres 22, 23 et 24 destinées au stockage ou à l'introduction des différents réactifs. La lame 25 de raclage du liquide contenu dans les fibres hydrophiles de la pastille 26 est montée radialement , solidaire de la partie supérieure du dispositif, entre les deux premières cavités 21 et 22.

Le dispositif comporte, sous la plaque de fond, un mécanisme d'entraînement non représenté de l'axe 19, qui est lui-même solidaire de la partie supérieure. Ce mécanisme, muni par exemple d'un remontoir, permet d'entraîner cette partie supérieure en rotation autour de l'axe 19 selon un séquencement déterminé. Ainsi, dans ce second mode de réalisation , le fonctionnement du dispositif n'est pas entièrement manuel, mais en partie automatique.

Le matériau plan hydrophobe est fixé sur sa plaque support et ce sont les cavités 21 à 24 et la lame 25 de raclage qui se déplacent au-dessus dudit matériau hydrophobe par rotation autour de l'axe 19. Le mécanisme est programmé pour que la vitesse de rotation corresponde aux temps de contact nécessaires d'une part à l'immobilisation des molécules déterminées sur le matériau hydrophile 26 et d'autre part à l'action des réactifs subséquents.

Dans le troisième mode de réalisation de l'invention, illustré par la figure 4, le support 27 est une boîte creuse composée d'une partie inférieure qui est une plaque 28 rigide et plane et d'une partie supérieure attenante qui est creusée d'une cavité 29 sensiblement centrale et qui comprend une lame rigide 30 intérieure et placée à proximité du bord comportant le long de la plaque 28 une fente 31.

Le matériau plan hydrophobe est, comme dans le premier mode de réalisation une bandelette 34 mais qui comporte en surface deux zones 31,32 présentant un matériau hydrophile d'immobilisation.

Avant utilisation, la première zone 32 est placée sous la cavité 29 contenant ou dans laquelle est introduit le milieu liquide à tester. Une fois que le liquide a été absorbé par le matériau hydrophile de la première zone 32 et que les molécules déterminées ont été immobilisées par ledit matériau , on fait coulisser la bandelette 34 en tirant sur l'extrémité 35 qui dépasse de la boîte 27 par la fente 31. Lors du déplacement de la bandelette, la première zone 32 passe sous la lame 30 qui racle le matériau hydrophile correspond et extrait mécaniquement le liquide qu'il contient et qui est exempt des molécules déterminées; le tirage se poursuivant, le liquide , par exemple sous forme d'une goutte 36 , comme cela est représenté sur la figure 4 , est maintenu par la lame 30 sur la surface de la bandelette 34 hydrophobe ; le tirage de la bandelette est interrompu lorsque le liquide 36 est au niveau de la seconde zone 33 d'immobilisation dans laquelle il est absorbé par le matériau hydrophile.

Dans ce cas , c'est la seconde zone 33 qui est le siège de la réaction nécessaire au test. Par exemple le matériau hydrophile de cette seconde zone 33 comporte des enzymes immobilisées capables de capter des molécules du liquide 36 après son passage sur la première zone 32, et de réaliser une réaction enzymatique décelable par exemple par une substance chromogène. Cette substance chromogène peut éventuellement être introduite dans la cavité 29 avec le milieu liquide à tester.

On a testé notamment le glucose dans le sang, avec dans la première zone 32 des fibres de viscose greffées dont les greffons sont porteurs de groupements sulfoniques, capables d'immobiliser les protéines, et dans la seconde zone 33 des fibres sur lesquelles est fixée la glucose oxydase, qui est une enzyme captant le glucose.

L'invention n'est pas limitée aux trois modes de réalisation qui ont été décrits, mais en couvre toutes les variantes.

La présentation du matériau hydrophile sous forme de fibres floquées dans un matériau hydrophobe est particulièrement avantageuse , du fait d'une grande surface spécifique des fibres ainsi disposées par rapport au milieu liquide , mais d'autres présentations peuvent être mises en oeuvre, par exemple sous forme de bouclettes , éponge etc...

Le matériau hydrophile a pour fonction d'immobiliser certaines molécules. Cette immobilisation est obtenue, en fonction du type de molécules par toute voie appropriée : liaisons covalentes , complexation, échange d'ions. Il reste à l'homme du métier de choisir les constituants dudit matériau hydrophile ou de réaliser les traitements adéquats pour obtenir cette immobilisation.

Parmi les traitements , on retiendra , en particulier la technique consistant à greffer sur la structure polymérique du matériau hydrophile un greffon porteur de groupements fonctionnels capables de capter les molécules déterminées. Le positionnement des groupements fonctionnels sur des greffons améliore l'accessibilité des molécules et la cinétique d'immobilisation des molécules.Par ailleurs,l'effet d'immobilisation est permanent du fait de l'attachement des groupements fonctionnels par liaison covalente.

L'invention voit ses applications préférées dans le domaine des tests hologiques , où l'on recherche des méthodes rapides et sûres de dosage et où un dispositif à usage unique présente bon nombre d'avantages ; cependant le dispositif peut être utilisé dans d'autres domaines , sans pour autant sortir du cadre de l'invention, tels que l'analyse chimique courante.

Par ailleurs, le dosage peut être effectué à partir des molécules immobilisées sur l'élément d'immobilisation , comme cela a été décrit ci-dessus ; mais peut aussi être effectué , sans sortir du cadre de l'invention, sur le milieu liquide dont on a extrait lesdites molécules et qui est évacué du matériau hydrophile par raclage. Dans ce cas, le dispositif comprend un moyen de récupération dudit milieu liquide, par exemple une cavité formée dans la plaque de fond en avant du moyen de raclage dans le premier mode de réalisation.

## Revendications

1. Dispositif à usage unique pour tests biologiques du type comportant au moins un élément d'immobilisation de molécules déterminées contenues dans un milieu liquide, caractérisé en ce que l'élément d'immobilisation est sous la forme d'un matériau hydrophile (1) fixé sur la surface d'un matériau plan hydrophobe (3) et en ce qu'il comprend :
a. une plaque de fond (5) supportant le matériau plan hydrophobe (3)
b. au moins une cavité (6) pour le milieu liquide à tester, dont l'orifice (9) d'évacuation est placé au-dessus du matériau plan hydrophobe
c. et un moyen de raclage (8) agissant sur le matériau hydrophile (1) et apte à déplacer le liquide que celui-ci contient sur la surface du matériau plan hydrophobe (3).

2. Dispositif selon la revendication 1 caractérisé en ce que l'élément d'immobilisation se présente sous la forme de fibres hydrophiles (2) implantées sensiblement verticalement dans le matériau plan hydrophobe (3).

3. Dispositif selon la revendication 2 caractérisé en ce que les fibres hydrophiles (2) sont des fibres de viscose, implantées par flocage dans une feuille de polychlorure de vinyle (3).

4. Dispositif selon la revendication 1 caractérisé en ce qu'il comprend une première cavité (6) pour le milieu liquide à tester et au moins une seconde cavité (7) pour un réactif, les cavités (6,7) étant placées au-dessus du matériau plan hydrophobe (3) de telle sorte que, par un déplacement relatif des cavités (6,7) et du matériau plan hydrophobe, le matériau hydrophobe puisse se trouver successivement sous l'orifice d'évacuation de la première (9) puis de la seconde cavité (13), et en ce que le moyen de raclage (8) agit sur le matériau hydrophile (1) après passage de celui-ci sous la première cavité (6).

5. Dispositif selon la revendication 4 caractérisé en ce que la plaque de fond (5) , les cavités (6,7) et le moyen de raclage (8) sont assemblés sous forme d'un boîtier (4) à l'intérieur duquel le matériau plan hydrophobe peut se déplacer par glissement sur la plaque de fond (5).

6. Dispositif selon la revendication 4 caractérisé en ce que d'une part la plaque de fond (5) et d'autre part les cavités (6,7) et le moyen de raclage (8) forment deux ensembles indépendants et en ce qu'il comprend un mécanisme d'entraînement provoquant le déplacement relatif de l'un de ces deux ensembles par rapport à l'autre.

7. Dispositif selon la revendication 5 caractérisé en ce qu'il a une forme globalement circulaire et en ce que le mécanisme d'entraînement provoque le déplacement de l'ensemble comprenant les cavités (6,7) et le moyen de raclage (8) en rotation autour de son axe (19) au-dessus de l'ensemble comprenant la plaque de fond (5).

8. Dispositif selon la revendication 1 caractérisé en ce que le moyen de raclage est une lame (8) rigide dont le bord inférieur (12) s'applique sur le matériau plan hydrophobe (3) supporté par la plaque de fond (5).

9. Dispositif selon la revendication 1 caractérisé en ce qu'il comporte au moins deux éléments d'immobilisation de molécules différentes, le premier étant placé sous la cavité (6) contenant le milieu liquide à tester et le second étant éloigné du premier d'un intervalle donné, et en ce que le moyen de raclage (8) est apte à déplacer le liquide , contenu dans le matériau hydrophile du premier élément d'immobilisation , sur la surface du matériau plan hydrophobe (3) le long de l'intervalle donné jusqu'au matériau hydrophile du second élément d'immobilisation.

10. Dispositif selon la revendication 1 caractérisé en ce que l'élément d'immobilisation comporte une structure polymérique sur laquelle sont greffés des greffons porteurs de groupements fonctionnels capables de capter les molécules déterminées.

11. Utilisation du dispositif selon la revendication 1 au dosage des immunoglobulines caractérisée en ce que l'élément d'immobilisation est à base de fibres cellulosiques greffées , dont les greffons comportent des fonctions sulfoniques.

12. Utilisation du dispositif selon la revendication 9 aux dosages biochimiques caractérisée en ce que le second élément d'immobilisation comporte une enzyme apte à développer une réaction enzymatique.

13. Utilisation selon la revendication 12 au dosage du glucose dans le sang caractérisée en ce que le premier élément d'immobilisation est apte à immobiliser les protéines et en ce que le second élément comporte de la glucose-oxydase.

## Patentansprüche

1. Vorrichtung zum einmaligen Gebrauch für biologische Tests des Typs, der mindestens ein Element zur Immobilisierung von in einem flüssigen Milieu enthaltenen, bestimmten Molekülen aufweist,
dadurch **gekennzeichnet**, daß
das Element zur Immobilisierung in Form eines auf der Oberfläche eines ebenen hydrophoben Materials (3) fixierten hydrophilen Materials (1) vorliegt und daß es enthält:
a) eine das ebene hydrophobe Material (3) tragende Trägerplatte (5),
b) mindestens eine Vertiefung (6) für das zu untersuchende flüssige Milieu, deren Entnahmeöffnung (9) oberhalb des ebenen hydrophoben Material angeordnet ist,
c) ein Mittel zum Abschaben (8), das auf dem hydrophilen Material (1) arbeitet und geeignet ist, die Flüssigkeit, die dieses an der Oberfläche des ebenen hydrophoben Materials (3) enthält, zu verschieben.

2. Vorrichtung nach Anspruch 1,
dadurch gekennzeichnet, daß
das Element zur Immobilisierung in Form hydrophiler Fasern (2) vorliegt, die im ebenen hydrophoben Material (3) ungefähr vertikal eingesetzt sind.

3. Vorrichtung nach Anspruch 2,
dadurch gekennzeichnet, daß
die hydrophilen Fasern (2) Viskosefasern sind, die durch Flockieren in einem Bogen aus Polyvinylchlorid (3) eingesetzt sind.

4. Vorrichtung nach Anspruch 1,
dadurch gekennzeichnet, daß
sie eine erste Vertiefung (6) für das zu untersuchende flüssige Milieu und mindestens eine zweite Vertiefung (7) für ein Reagenz aufweist, wobei die Vertiefungen (6, 7) oberhalb des ebenen hydrophoben Materials (3) so angeordnet sind, daß durch eine relative Verschiebung der Vertiefungen (6, 7) und des ebenen hydrophoben Materials sich das hydrophobe Material nacheinander unter der Entnahmeöffnung der ersten (9) und anschließend der zweiten (13) Vertiefung befinden kann, und daß das Mittel zum Abschaben (8) auf dem hydrophilen Material (1) arbeitet, nachdem dieses unter der ersten Vertiefung (6) vorbeigezogen ist.

5. Vorrichtung nach Anspruch 4,
dadurch gekennzeichnet, daß
die Trägerplatte (5), die Vertiefungen (6, 7) und das Mittel zum Abschaben (8) in Form einer Dose (4) zusammengesetzt sind, in deren Inneren sich das ebene hydrophobe Material durch Gleiten auf der Trägerplatte (5) bewegen kann.

6. Vorrichtung nach Anspruch 4,
dadurch gekennzeichnet, daß
einerseits die Trägerplatte (5) und andererseits die Vertiefungen (6, 7) und das Mittel zum Abschaben (8) zwei voneinander unabhängige Einheiten bilden und daß sie einen Mechanismus zur Mitnahme enthält, die die relative Verschiebung einer der beiden Einheiten in bezug auf die andere bewirkt.

7. Vorrichtung nach Anspruch 5,
dadurch gekennzeichnet, daß
sie eine im ganzen kreisförmige Form besitzt und daß der Mechanismus zur Mitnahme die Verschiebung der die Vertiefungen (6, 7) und das Mittel zum Abschaben (8) enthaltenden Einheit durch Rotation um seine Achse (19) oberhalb der die Trägerplatte (5) enthaltenden Einheit bewirkt.

8. Vorrichtung nach Anspruch 1,
dadurch gekennzeichnet, daß
das Mittel zum Abschaben ein starres Blatt (8) ist, dessen unterer Rand (12) auf dem von der Trägerplatte (5) getragenen ebenen hydrophoben Material aufliegt.

9. Vorrichtung nach Anspruch 1,
dadurch gekennzeichnet, daß
sie mindestens zwei Elemente zur Immobilisierung von verschiedenen Molekülen enthält, wobei das erste unter der Vertiefung (6) angeordnet ist, die das zu untersuchende flüssige Milieu enthält, und das zweite vom ersten in einer gegebenen Entfernung beabstandet ist, und daß das Mittel zum Abschaben (8) geeignet ist, die im hydrophilen Material des ersten Elements zur Immobilisierung enthaltene Flüssigkeit auf der Oberfläche des ebenen hydrophoben Materials (3) entlang der gegebenen Entfernung bis zum hydrophilen Material des zweiten Elementes zur Immobilisierung zu verschieben.

10. Vorrichtung nach Anspruch 1,
dadurch gekennzeichnet, daß
das Element zur Immobilisierung eine polymere Struktur aufweist, auf die Pfropfeinheiten aufgepfropft sind, die zum Abfangen der bestimmten Moleküle geeignete funktionelle Gruppen enthalten.

11. Verwendung der Vorrichtung nach Anspruch 1 zur Dosierung von Immunglobulinen,
dadurch gekennzeichnet, daß
das Element zu Immobilisierung auf der Basis gepfropften Cellulosefasern ist, deren Pfropfeinheiten Sulfonsäuregruppen enthalten.

12. Verwendung der Vorrichtung nach Anspruch 9 zu biochemischen Dosierungen,
dadurch gekennzeichnet, daß
das zweite Element zur Immobilisierung ein Enzym enthält, das geeignet ist, eine enzymatische Reaktion zu bewirken.

13. Verwendung nach Anspruch 12 zur Dosierung von Glucose in Blut,
dadurch gekennzeichnet, daß
das erste Element zur Immobilisierung zur Immobilisierung von Proteinen geeignet ist und daß das zweite Element Glucoseoxidase enthält.

## Claims

1. One-use device for biological tests of the type comprising at least one element for immobilizing specific molecules contained in a liquid medium, characterized in that said immobilizing element is in the form of a hydrophilic material (1) fixed on the surface of a plane hydrophobic material (3), and in that it comprises:
a) a bottom plate (5) supporting the plane hydrophobic material (3),
b) at least one cavity (6) for the liquid medium to be tested, said cavity having an outlet orifice (9) situated above the plane hydrophobic material, and
c) scraping means (8) acting on the hydrophilic material (1) and capable of moving the liquid contained therein over the surface of the plane hydrophobic material (3).

2. Device according to claim 1, characterized in that the immobilizing element is in the form of hydrophilic fibers (2) implanted substantially vertically in the plane hydrophobic material.

3. Device according to claim 2, characterized in that the hydrophilic fibers (2) are viscose fibers, implanted by flocking in a sheet of polyvinyl chloride (3).

4. Device according to claim 1, characterized in that said device comprises a first cavity (6) for the liquid medium to be tested and at least a second cavity (7) for a reagent, said cavities (6, 7) being situated above the plane hydrophobic material (3) so that, by a relative displacement of the cavities (6, 7) and of the plane hydrophobic material, the hydrophilic material can be placed, successively, under the outlet orifice (9) of the first cavity and then under that (13) of the second cavity, and the scraping means (8) acts on the hydrophilic material (1) after said material has passed under the first cavity (6).

5. Device according to claim 4, characterized in that the bottom plate (5), the cavities (6, 7) and the scraping means (8) are assembled in the form of a box (4) inside which the plane hydrophobic material can move by sliding over the bottom plate (5).

6. Device according to claim 4, characterized in that on the one hand the bottom plate (5) and on the other hand the cavities (6, 7) and the scraping means (8) form two independent assemblies and in that it comprises a driving mechanism causing the relative displacement of one of said two assemblies with respect to the other.

7. Device according to claim 5, characterized in that the device has an overall circular shape and in that the driving mechanism drives the assembly composed of the cavities (6, 7) and the scraping means (8), in rotation about its axis (19), above the assembly comprising the bottom plate (5).

8. Device according to claim 1, characterized in that the scraping means is a rigid plate (8) of which the lower edge (12) is applied on the plane hydrophobic material (3) supported by the bottom plate (5).

9. Device according to claim 1, characterized in that said device comprises at least two immobilizing elements, each element immobilizing different molecules, the first element being placed under the cavity (6) containing the liquid medium to be tested, and the second element being at a given distance from the first, and the scraping means (8) is capable of moving the liquid contained in the hydrophilic material of the first immobilizing element, over the surface of the plane hydrophobic material (3) along the given distance up to the hydrophilic material of the second immobilizing element.

10. Device according to claim 1, characterized in that the immobilizing element comprises a polymeric structure on which are grafted grafts which are carriers of functional groups capable of sensing the specific molecules

11. Use of the device according to claim 1 for assaying immunoglobulins, characterized in that the immobilizing element is based on grafted cellulosic fibers, of which the grafts contain sulfonic functions.

12. Use of the device according to claim 9 in biological assays, characterized in that the second immobilizing element comprises an enzyme capable of producing an enzymatic reaction.

13. Use of the device according to claim 12 for assaying glucose in the blood, characterized in that the first immobilizing element is capable of immobilizing the proteins while the second element contains glucose oxidase.
